# EUROPÄISCHE PATENTSCHRIFT

(11) **EP 1 633 857 B1**
(45) Veröffentlichungstag und Bekanntmachung des Hinweises auf die Patenterteilung: **23.07.2008**
(21) Anmeldenummer: 04738577.8
(22) Anmeldetag: 27.05.2004
(51) Int. Cl.: C12N 1/04, B65D 83/14, A61M 1/00

(54) **HYGIENISCHE SICHERUNG DER MEHRKAMMERDOSIERSPRÜHTECHNIK UND DEREN PROBIOTSCHE ANWENDUNG ZUR APPLIKATION LEBENSFÄHIGER MIKROORGANISMEN**
HYGIENIC CONSOLIDATION OF A MULTI-CHAMBER DOSING SPRAY TECHNIQUE AND THE PROBIOTIC USE THEREOF FOR THE APPLICATION OF VIABLE MICRO-ORGANISMS
SECURISATION HYGIENIQUE DU PROCEDE DE PULVERISATION DE DOSAGE A PLUSIEURS CHAMBRES ET UTILISATION PROBIOTIQUE POUR L'APPLICATION DE MICRO-ORGANISMES VIABLES

(30) Priorität: 05.06.2003 DE 10325404
(43) Veröffentlichungstag der Anmeldung: 15.03.2006
(73) Patentinhaber: Thenn, Willy, 82377 Penzberg (DE); Lauer, Eckhard, Dr., D-12249 Berlin (DE)
(72) Erfinder: Thenn, Willy, 82377 Penzberg (DE); Lauer, Eckhard, Dr., D-12249 Berlin (DE)
(74) Vertreter: Flosdorff, Jürgen
(86) Internationale Anmeldenummer: PCT/DE2004/001108
(87) Internationale Veröffentlichungsnummer: WO 2004/108905

(56) Entgegenhaltungen:
- DE-A- 2 852 779
- GB-A- 1 449 606
- US-A- 4 518 696
- US-A- 4 874 707
- DATABASE CA [Online] CHEMICAL ABSTRACTS SERVICE, COLUMBUS, OHIO, US; MATSUBARA, YUIKO ET AL: "Potent antagonistic effects of probiotics , Enterococcus faecalis and Clostridium butyricum, for methicillin-resistant Staphylococcus aureus" XP002305583 gefunden im STN Database accession no. 132:331842 & IGAKU TO SEIBUTSUGAKU , 139(4), 185-190 CODEN: IGSBAL; ISSN: 0019-1604, 1999,
- PARAJE M G ET AL: "Conservation in probiotic preparations of Lactobacillus with inhibitory capacity on other species" MICROBIOLOGICA (PAVIA), Bd. 23, Nr. 4, Oktober 2000 (2000-10), Seiten 423-431, XP009039893 ISSN: 1121-7138
- HASSIEN ET AL: "Nutritional Requirements of L. Bifidus" J. BACTERIOL., Bd. 6, 1951, Seiten 771-777,

## Beschreibung

Probiotische Mittel ["Probiotics" von *pro* (lat.) und *bios* (griech.) soviel wie "nützlich für das Leben"] sind nach Fuller (J. Appl. Bacteriol. 66, 365-378,1989) solche, die spezielle Mikroorganismen, z. B. als Nahrungssupplement, enthalten und den Zweck haben, die intestinale, physiologische Balance im Darm bzw. dessen mikrobiologisches Ökosystem, die "Biofilmbesiedlung" auf den Schleimhäuten, gesundheitlich zu unterstützen. Heutzutage wird diese Definition sogar nicht nur auf den Darm, sondern sinngemäß und im weiteren Sinn auf die Biofilmbesiedlungen der Haut oder der Schleimhaut (Mund, Rachen, Nase, Vagina) bezogen. Im Darm sind gesundheitlich positive Effekte metabolisch und/oder immunologisch meßbar und werden wissenschaftlich kategorisiert als "mikrobiell assoziierte Charakteristiken (MAC)" (Luckey: Am, J. Clin. Nutr. 30, 1753-1761, 1977; Midtvedt et al.: J. Ped. Gastroenterol. Nutr. 7, 559-567, 1988; Fuller, Cole: Chapter 1 in: Probiotics-Theory and Applications. Stark, Wilkinson (eds.), Chalcombe Publ., 1989). Diese "symbiotischen" Beziehungen zwischen Wirt und Bakterienbesiedlung sind lebenslang wesentlich für die Gesundheit und für die Lebensfähigkeit in normaler Umwelt überhaupt.

Probiotische Produkte, z. B. Joghurt mit Zusatz spezieller Kulturen, sind lange bekannt und wurden erfolgreich auch schon vor Bekanntwerden des unspezifizierten Begriffs "probiotisch" vermarktet. Auch Medikamente, die spezielle, lebensfähige Kulturen und/oder deren Bestandteile und Stoffwechselprodukte als Wirkstoffe enthalten, wurden entwickelt (Mackowiak: Am. J. Med. 67, 293-306, 1979; Salminen, von Wright: Lactic Acid Bacteria. Marcel Dekker, Inc. Basel, 1993). Die heute vorrangig als "Nahrungssupplement" vermarkteten Milchprodukte basieren meistens auf flüssiger Mediumgrundlage, Medikamente dagegen auf gefriergetrockneten Kulturen, dosiert in Tabletten oder Kapseln. Die Anwendung solcher probiotischen Kulturen erfolgt also entweder in frisch kultivierter, sozusagen Joghurt-ähnlicher, flüssiger oder in gefrier-, sprüh- oder wirbelschichtgetrockneter Form. Die direkte Auftragung von Mikroorganismen mittels "Kontaminationssprühnebel" auf die Haut oder Schleimhaut ist nur selten versucht worden, denn diese Anwendungsform birgt noch zu viele Probleme und Risiken, wie weiter unten näher ausgeführt wird.

Für Darmzubereitungen werden bisher ausschließlich als apathogen bezeichnete Bakterien, meist Milchsäurebakterien mit der niedrigsten Erregerrisikoeinstufung genutzt, wie Laktobazillen, Laktokokken, Enterokokken oder Bifidobakterien, aber auch apathogene Hefen, z. B. "*Saccharomyces boulardii*"*.* Eine wissenschaftlich und klinisch fundierte Begründung für solche Wirkstoffe ist nur selten gegeben (Hooper et al.: Ann. Rev. Nutr. 22, 283-307, 2002; Mercenier et al.: Curr. Pharmaceut. Design 9, 175-191, 2003). Die *"ratio"* zwischen Anwendungszweck und Art bzw. Eigenschaften der verwendeten Mikroorganismen ist meist vage begründet.

Statt probiotischer Komponenten kommen alternativ oder in "synbiotischer" Kombination mit diesen auch "prebiotische" Stoffe zum Einsatz. Hierbei handelt es sich um Substanzen, die die metabolische Aktivität einer Biofilmbesiedlung indirekt fördern sollen. Lange bekannt ist z. B. die Anwendung von Lactulose, ein Zucker, der nicht im menschlichen Dünndarm absorbiert, wohl aber durch Biofilm-Bakterien metabolisiert werden kann und somit deren Aktivität fördert (Fuller, Cole, 1989). Dadurch wird der Ammoniak-Spiegel im Blut bei Leberzirrhose-Patienten gesenkt.

Aufgrund der beschränkten Kenntnis der komplexen, physiologischen Interaktionen von Wirtsorgan und Biofilmgemeinschaft, war die Anwendung lebensfähiger Bakterien als Wirkstoffe bisher praktisch nur zur oralen oder vaginalen Applikation entwickelt. Dabei hatte zunächst eine Unterscheidung von Wirkstoffen in flüssiger oder getrockneter Form keine primäre Bedeutung, allenfalls eine der qualitativen Art. So sind flüssige Kulturen i. d. R. von labiler Qualität, anders als gefriergetrocknete Präparationen. Umgekehrt ist ein Nachteil der trockenen Ausbringung die eventuell ungleichmäßige Verteilung des Wirkstoffs auf der Schleimhaut, wodurch ein günstiger Effekt erheblich limitiert sein kann. Eine Anwendung auf empfindlichen Schleimhäuten, wie des Rachens, der Nase, der Bronchien oder im Augenbereich wäre so überhaupt nicht zu verwirklichen.

Zur Ausweitung der Anwendungsmöglichkeiten bestand also die Aufgabe, ein System zu haben, das die Vorteile der flüssigen und getrockneten Form des kulturellen Wirkstoffs vereint und somit auch kritischere Anwendungen erlaubt. Dies wäre z. B. mit einem Dosierspray erreichbar, dessen Dosierflüssigkeit, nach Suspendieren eines Trockenkulturpulvers mit einem Flüssigmedium kurz vor Anwendung, eine fein dosierbare, homogene Suspension ist. Dies und einige Dosiergeräte sind dem Fachmann durchaus schon bekannt, und dieser kennt auch das inhärente Problem dieser Technik, nämlich das Risiko der hygienischen Instabilität von flüssigen Zubereitungen, die häufig auftretenden Fremdkontaminationen während des Anwendungszeitraums. Aus diesem Grund hat sich praktisch für Dosiersysteme die Beigabe von Konservierungsmitteln in der Suspension als unabdingbar eingebürgert. Dies schützt den Patienten vor einem möglichen Infektionsrisiko und den Dosiersysteminhalt vor dem Verderb. Viele Konservierungsmittel haben leider besondere Probleme: allergische oder toxische Reaktionen beim Patienten, kurze Überlebensraten des probiotischen Wirkstoffs und immer noch weiter bestehende hygienische Risiken durch Kontamination mit resistenten Keimen.

Zur Vermeidung von Konservierungsstoffen bei Dosiersystemen sollten folgende Voraussetzungen erfüllt sein: (i) Der Inhalt sollte durch einen entsprechend präzise gearbeiteten Verschluß vor Kontamination dauerhaft geschützt sein. (ii) Der beim Sprühstoß entstehende Unterdruck sollte so ausgeglichen werden, dass keine kontaminierte Luft in die Zubereitung gelangt. (iii) Auch im Bereich des Auslassventils, durch das die Zubereitung abgegeben wird, sollten in Flüssigkeltsresten - weder durch eingesaugte, kontaminierte Luft noch durch Diffusion von außen - Kontaminationen auftreten.

Ein Dosiersystem, das diese Voraussetzungen erfüllen kann, ist in der DE 19742559 C2 offenbart.

Es ist bekannt, dass Silber im Kontakt mit Wasser geringe Mengen an Silber-Ionen (Ag⁺) bildet, die allgemein antibakteriell wirken, was als ollgo-dynamischer Effekt bezeichnet wird.

US-A-4 874 707 offenbart ein Verfahren zur Herstellung einer wäßrigen Suspension von nitrifizierenden Bakterien. Dabei handelt es sich im Gegensatz zu den Milchsäurobekterlen um Gram-negative Bakterien mit strikt areobem Wachstumsanspruch, die nicht in der Lage sind, in einem üblichen Medium für Milchsäurebakterien zu gedeihen.

Beschrieben wird ein Lyophilisat mit lebensfähigen Mikroorganismen, Bestandteilen oder Stoffwechselprodukten derselben das in Form ei-ner Suspension in einem aktivitätserhaltenden und konservierenden Medium vorliegt und als Wirkstoff zur aktiven Besinflussung oder Generierung mikrobieller Biofilmgesellschaften auf Menschen, Tiere oder Pflanzen oder Organe derselben mit Hilfe eines Dosiersprühnebels in gleichmäßiger Vertellung aufsprühbar ist.

Weiter ist vorgesehen, daß es sich bei den Mikroorganismen um Bakterien oder deren Bestandteile oder Stoffwechselprodukte handelt. Bevorzugt handelt es sich bei den Bakterien um probiotische Bakterien, Insbesondere Milchsäurebakte-rien.

Das Lyophilisat ist als Wirkstoff zur metabolisch positiven Aktivierung menschlicher, tierischer oder pflanzlicher Organe verwendbar.

Die Erfindung sieht die Verwendung eines wässrigen Suspensionsmediums vor, das peptidische Bestandteile, insbesondere Pepton, und/oder wenigstens eine Aminosäure enthält, wobei das Supensionsmedium einen Gesamtstickstoffgehalt nach Kjeldahl von 0,01 bis 0,2% (m/v) hat, zur Aktivitätserhaltung und zur Konservierung von lebensfähigen Milchsäurebakterien für probiotische Anwendungen.

Die Erfindung sieht demnach ein Medium vor, das so wirkt, daß die probiotische Flüssigzubereitung nicht nur vor Fremdkontamination, sondern auch vor Inaktivierung wirksam geschützt ist. Die Effizienz ist nachweisbar anhand der stabilen Aktivität und Lebensfähigkeit der probiotischen Bakterien und der gleichzeitig bestehenden hygienischen Stabilität der Suspension während des Anwendungszeitraums. Die Besonderheit der Erfindung liegt darin, daß sich die gesteuerte Passivierung von Ag⁺ im Gemisch durch den Gehalt von Aminosäuren oder am einfachsten - weil Anminosäuren kostspielig sind bzw. weil ohnehin ein unspezifisches Gemisch von Aminosäuren und Peptiden genügt - durch bestimmten Gehalt von Pepton im Medium ersetzen läßt, obwohl gerade solche Bestandteile eher zur Vermehrung von Mikroorgamismen, also auch zur Förderung von Kontaminanten, geeignet sind.

Pepton, ein Gemisch aus freien Aminosäuren und Oligopeptiden, ist für auxotroph wachsende Bakterien, insbesondere für Milchsäure bildende Bakterien, z. B. der *Acidophilus*-Artengruppe, ein wesentlicher Kulturbestandteil. Pepton aus pflanzlichen Ausgangsmaterialien wie Soyabohnen oder aus tierischen Materialien wie Milch, Fleisch oder Leber erwiesen sich als vielseitig geeignet. Pepton wird z. B. durch kontrollierte enzymatische Proteolyse mit Pepsin unter sauren Bedingungen gewonnen. Die so erhaltene, aufgereinigte, sprühgetrocknete Substanz ist ein feines, hellbraunes und hygroskopisches Pulver mit charakteristischem, jedoch nicht fauligem Geruch, ist in Wasser gut löslich, unlöslich in Ethanol und Ether. Folgende chemisch-physikalischen Spezifikationen werden gemäß *Peptic Digest of Animal Tissue* USP zur Qualitätscharakterisierung geprüft: Grad des proteolytischen Abbaus, ggf. auch Rest an coagulierbarem Eiweiß, Gehalt an Gesamtstickstoff und Aminostickstoff, Trocknungsverlust, Sulfatasche-Menge, Nitritgehalt, pH-Wert der wässrigen Lösung und bakteriologisch-kulturelle Parameter. Je nach Ausgangsmaterial oder Herstellungsart sind erhebliche Toleranzen zu beachten: Gesamtstickstoff (Kjeldahl), 5-15 %m/m bzw. Aminostickstoff (betreffend Aminosäuren und Peptide), 1-6 %m/m; Sulfatasche, 2-15 %m/m; pH-Wert in 2%-iger Lösung: pH 6-8. **Anlage 1** zeigt die durchschnittliche Aminosäure-Zusammensetzung verschiedener Peptone (Angaben nach Merck AG, Darmstadt).

Spezifizierte Angaben für unsere Versuche.

Ein hoher Gesamtstickstoffgehalt von > 10 %m/m und Aminostickstoff von > 2 %m/m sind ein gutes Maß für unsere Zwecke, wobei dies prinzipiell keine Anforderung für einen bestimmten Gehalt individueller Aminosäuren voraussetzt. Denn es ist bekannt, daß zur Stabilisierung der Vermehrungs- und Lebensfähigkeit von Bakterien in wässriger Suspension das Vorliegen eines "Gesamt-Pools" von bis zu 20 Aminosäuren notwendig ist. Außerdem ist die kontrollierte Passivierung von Silber-lonen im Medium ebenfalls unabhängig von der Zusammensetzung bezüglich individueller Aminosäuren. Jedoch zeigten unsere Versuche, daß der Gehalt an Aminosäuren zur Passivierung beachtet werden muß, da eine direkte Korrelation besteht. Zum Beispiel weist das experimentell verwendete Pepton Oxoid L34 einen Gesamtstickstoffgehalt von ca. 14 %m/m auf, d. h. auf Medium A (zu starke Passivierung) mit 1 % Pepton/Lösung kommen 0,14 %m/m, auf Medium B (zielgerechte Passivierung) mit 0,1 % Pepton/Lösung kommen 0,014 %m/m und auf Medium C (höhere Passivierung als in Medium B) mit 0,5 % Pepton/Lösung kommen 0,07 %m/m Gesamtstickstoff allein aus Pepton; der Anteil Aminosäuren aus Hefe-Extrakt kann bei diesen Medien vernachlässigt werden, da er' nur geringe Mengen an freien Aminosäuren beträgt. Somit ergibt sich eine auf Gesamtstickstoff basierte Toleranz von 0,014-0,14 % für zielgerechte Passivierung von Ag⁺ in Medium.

Zum gleichen Zweck - was wir nicht getestet haben - können selbstverständlich auch andere Aminogruppen enthaltende Verbindungen anstelle von Aminosäuren verwendet werden, z. B. Ammoniumsalze, organische Ammoniumverbindungen, Guanido- oder Guanidino-Strukturen, Guanin-, Adenin-, Thymin- und Cytosin-basierte Strukturen, die in Medien für bakteriologische Zwecke verwendet werden. Derartige Verbindungen sind i.d.R. ohnehin in Pepton enthalten, wenn auch in substantiell vergleichsweise viel geringerer Konzentration als Aminosäuren.

Als probiotische Bakterien zur Anwendung beim Menschen oder Tier kommen vorrangig Milchsäurebakterien unterschiedlichster Art zur Anwendung, wie sie in der allgemeinen Literatur, z. B. *Section* 14 und 15 von Bergey's Manual of Systematic Bacteriology (Vol. 2, Sneath et al.; Williams & Wilkins, London 1986), beschrieben sind. Als Beispiel für unsere Versuche dienten Stämme von *Lactobacillus gasseri* (Section 14) und *Bifidobacterium longum* (Section 15), z. B. *B. longum* ATCC 15707.

Analog ist auch eine Sprühdosierung mit nicht lebensfähigen Bestandteilen zur Anwendung vorgesehen, z. B. mit Gram-negativen Bakteriengruppen, wie sie in Vol. 1 von Bergey's Manual of Systematic Bacteriology (Krieg und Holt (eds.), Williams & Wilkins, London 1984) beschrieben sind, z. B. *Acinetobacter calcoaceticus* ATCC 23055 (Section 4).

Analog ist auch eine Sprühdosierung mit lebensfähigen Bakterien an Pflanzen vorgesehen, z.B. mit Stickstoff-fixierenden Bakterien, etwa *Azotobacter*-Arten (Section 4), etwa *Azotobacter chroococcum* ATCC 480.

Analog ist eine Sprühdosierung von Pflanzen gegen Schadinsekten vorgesehen mit Bakterien der Gattungen *Sporolactobacillus* oder *Bacillus* (Section 13 in Vol. 2 von Bergey's Manual of Systematic Bacteriology), z. B. *Bac. thuringiensis* ATCC 10792.

### Zielgerichtete Effekte der Erfindung.

Wirkweisen der erfindungsgemäßen Suspension von lebensfähigen Bakterien in Medium bzw. des Mediums allein sind:
- der Pepton- bzw. Aminosäuregehalt des Mediums passiviert Ag⁺ ist so weit fixiert bzw. als Mediumbestandteil minimiert, daß die Entwicklung unerwünschter Keime gehemmt ist, während probiotische Bakterien ausreichend lange in lebens- und vermehrungsfähigem Zustand verbleiben
- das Vermischen des zunächst sterilen Mediums in der unteren Kammer des Mehrkammer-systems mit dem Kontaminanten-arm (=Fremdkeimarm) hergestellten Lyophilisat, enthaltend die probiotische Kultur, aus der oberen Kammer bedingt einen metastabilen Hygienezustand, der jedoch
   a) durch die besondere Zusammensetzung des Mediums allein schon ein Überwuchern mit Kontaminaten verhindert und
   b) gleichzeitig die Lebensfähigkeit der probiotischen Kultur ausreichend stabil erhält
- etwa in die Kultursuspension während der Anwendungsphase aus der Aussprühöffnung gelangte Ag⁺ hemmen die Aktivität der probiotischen Keime nicht, tragen dennoch zur Hemmung der Entwicklung fremder Keime bei
- die probiotischen Mikroorganismen üben von sich aus (auch ohne Ag⁺) einen Effekt gegen eine Kontaminationsentwicklung in der Suspension aus

Beschrieben wird ferner ein Zweikammer-Dosierspray-System, das eine chemische Konservierung aufgrund des "oligodynamischen Effekts" von Silber (Ag) erübrigt. Bei dem Dosiersystem befindet sich ein Silberfaden direkt im Sprühdosierkopf der Dosierpumpe, so daß von außen eingedrungene Fremdkeime durch den Kontakt mit Ag⁺ rasch inaktiviert und dadurch der flüssige Systeminhalt vor Kontamination geschützt werden. Anstelle eines Silberfadens kann auch eine Silberbeschichtung entlang der Austrittsbahn der Suspension vorgesehen sein. Silber kann zudem auch durch ein anderes oligodynamisch wirksames Metall ersetzt sein. Die Konstruktion des Mehrkammersystems garantierte zudem eine ausreichende Isolation des Kulturlyophilisats gegenüber Feuchte und Luft, indem außer einer Kammer für die Flüssigzubereitung mit dem Medium noch eine abgetrennte Kammer zur Aufnahme des probiotischen Kulturlyophilisats vorhanden ist. Diese Kammer ist mit dem Kulturlyophilisat befüllt, welches erst direkt vor der ersten Anwendung aseptisch mit dem Medium in der Mediumkammer vermischt wird, um so eine frisch zubereitete Kultursuspension zu erzielen. Hierzu wird der Boden der Kammer mittels der in die Funktionsstellung vorgeschobenen *Airless-*Pumpe des Systems geöffnet. Diese Lyophilisatkammer ist absolut vor Luft und Feuchtigkeit von außen und aus der Mediumkammer geschützt, um eine konstante Qualität des Kulturlyophilisats und der probiotischen Wirksamkeit auch während der Lagerhaltung zu gewährleisten. Gleichzeitig ist das Risiko von Kontaminationen unterbunden.

Weiter ist vorgesehen, daß die Kammer, die die Suspension aufnimmt, vorteilhaft z.B. durch einen weichen Beutel gebildet ist, der sich bei Abgabe des Kammerinhalts zusammenzieht. Der weiche Innenbeutel befindet sich bevorzugt innerhalb eines weitgehend starren Außenbehälters, der Öffnungen zum Eintritt von Luft in den Zwischenraum zwischen Innenbeutel und Außenbehälter hat. Der Sprühdosierkopf der Dosierpumpe ist bevorzugt so ausgebildet, daß keinerlei Luft von außen in den Innenbeutel eintreten kann.

Die Ausbildung kann aber auch so getroffen sein, daß die die Suspension aufnehmende Kammer durch einen starren Behälter gebildet ist, in den nach Abgabe von Flüssigkeit jeweils Luft zum Druckausgleich eintritt, die dabei aber einen geeigneten Filter durchströmen muß.

Beschrieben wird somit ein Lyophilisat mit lebensfähigen Mikroorganismen, Bestandteilen oder Stoffwechselprodukten derselben das in Form einer Suspension in einem aktivitätserhaltenden, revitalisierenden und konservierenden Medium vorliegt, das eine oder mehrere Aminosäuren und/oder peptidische Bestandteile, insbesondere Pepton, enthält, wobei die Aminosäure(n) und/oder peptidischen Bestandteile in einer Menge von bis zu 0,2% (m/v) bezogen auf den Gesamtstickstoffgehalt des Suspensionsmediums enthalten sind.

Demnach wird eine stabile Suspension von Mikroorganismen, insbesondere Milchsäurebakterien, beschrieben mit dem Effekt der vollen Revitalisierung und Aktivitätserhaltung aus einem bevorzugt gefriergetrockneten Zustand bei gleichzeitiger Sicherung des hygienischen Reinheitszustands der Suspension.

Die Erfindung sieht ferner ein Zweikammer-Dosierabgabesystem vor, mit einer ersten Kammer, die in einem Ausgangszustand eine Suspensionsflüssigkeit enthält, einer zweiten Kammer, die in einem Ausgangzustand in trockener Form vorliegende Milchsäurebakterien enthält und luftdicht verschlossen ist, und mit einer Dosierpumpe, wobei vor Ingebrauchnahme des Systems die zweite Kammer zur ersten Kammer hin geöffnet wird, so daß die Milchsäurebakterien in die Suspensionsflüssigkeit gelangen, wobei die Suspensionsflüssigkeit eine oder mehrere Aminosäuren und/oder peptidische Bestandteile, insbesondere Pepton enthält, und wobei die Suspensionsflüssigkeit einen Gesamtstickstoffgehalt nach Kjeldahl von 0,01 ein 0,2% (m/v) hat.

Das erfindungsgemäße Zweikammer-Dosierabgabesystem gewährleistet eine hygienische Spraydosierung der Suspension ohne Konservierungsmittel vor und während des Anwendungszeitraums.

In den folgenden Versuchen wird das Verhalten unterschiedlicher Bakterien in von uns entwickelten Suspensionsmedien unterschiedlicher Zusammensetzung vergleichend dargestellt und der Passivierungsfaktor veranschaulicht. Dem Fachmann geläufig ist die Herstellung von Medien, die Handhabung der Kulturen, die Feststellung des aktiven Zustands einer Kultur anhand der Prüfung des Koloniebildungsvermögens der Testkeime auf geeigneten Spezial-medien. Außer unserer probiotischen Kultur wurden auch solche Kontaminationskeime getestet, die der Fachmann zur Prüfung auf ausreichende Konservierung einsetzt (Bagel, Wiedemann: DAZ 139, 4438-4441, 1999). Zusätzlich wurden diese Testkeime mit und ohne Beimischung unserer probiotischen Kultur, also auch im Kulturgemisch, untersucht sowie zum Vergleich das Verhalten in unterschiedlich zusammengesetzten Medien.

### Experimente.

### a) Zusammensetzung und Herstellung der Suspensionsmedien:

**Tabelle 1: Tabellarische Darstellung der Zusammensetzung der Suspensionsmedien.**

| **Substanz *)** (bzw. Bedingung): | **Zusammensetzung des Mediums (g/Liter):** | | | | |
|---|---|---|---|---|---|
| | **Medium A** | **Medium B** | **Medium C** | **Medium D** | **Medium E** |
| Fleischpepton | 10 | 1 | 5 | 0 | 0 |
| Hefeextrakt | 1 | 0 | 0,5 | 0 | 0 |
| Natriumacetat | 3 | 3 | 3 | 0 | 0 |
| Natriumchlorid | 5 | 4 | 5 | 0 | 8 |
| Magnesiumsulfat | 0,2 | 0 | 0 | 0 | 0 |
| Kaliumphosphat | 0,8 | 1 | 0,8 | 0 | 0 |
| Natriumascorbat | 0 | 0 | 0 | 8 | 0 |
| Ascorbinsäure | 0 | 0 | 0,8 | 0 | 1 |
| Tween 80 | 0,2 | 0,2 | 0,2 | 0,2 | 0,2 |
| pH-Wert : | 6,0 | 5,9 | 4,8 | 6,5 | 3,1 |

| | | | | | |
|---|---|---|---|---|---|
| *) Näheres, s. Text. | | | | | |

Als Substanzen kommen üblicherweise auf dem Markt verfügbare Materialien zum Einsatz, etwa Fleischpepton *Oxoid* L 34, Hefeextrakt *Oxoid* L 21, Na-Acetat *Merck* Art. 6265, Na-Chlorid *Merck* Art. 6400, Mg-Sulfat *Merck* Art. 5882, Kaliumdihydrogensulfat *Merck* Art. 104873, Tween 80 *Merck* Art. 822187, Na-Ascorbat *Merck* Art. 500076, Ascorbinsäure *Merck* Art. 500074. Die Substanzen werden in entionisiertem Wasser (destilliertem Wasser) zu 1000 ml gelöst und der pH-Wert mit einem pH-Meßgerät geprüft. Die Verteilung der Medien zu 15 ml in sterile Proben- bzw. Dosierspraybehältnisse erfolgt aseptisch bei gleich-zeitiger Sterilfiltration mittels Membranfiltration (Membran: Nitrocellulose/50 mm Durchmesser/0,2 Mikrometer Porenweite/steril) oder ggf. durch Verteilen nach Autoklavieren des Mediums bei 121°C für 15 min.

### b) Art und Stammbezeichnung der Testorganismen:

*Escherichia coli* ATCC 8739, *Pseudomonas aeruginosa* ATCC 9027, *Staphylococcus aureus* ATCC 6538P, *Candida albicans* ATCC 10231, *Aspergillus niger* ATCC 16404, *Lactobacillus gasseri* ATCC 33323. As probiotische Testkultur dient z. B. ein Stamm *L. gasseri* H18b, beispielsweise als gefriergetrocknete Präparation mit mind. 10⁸ KBE/g in einem Stoffgemisch aus Bakterien, Kulturbestandteilen, Zuckern, Gelatine und Ascorbat und mit einer max. Restfeuchte von 2 %. Der Einfluß solcher Zusätze muß grundsätzlich bei der Passivierung von Ag⁺ auch berücksichtigt werden. Wenn ihr Anteil im fertig suspendierten Stoffgemisch allerdings - wie hier in unserem Fall - vergleichsweise minimal ist, kann dies i.d.R. vernachlässigt werden; in unserem Testgemisch zeigte sich kein Effekt.

### c) Kulturbedingungen:

Die Kulturbedingungen und Standardmedien für die Testorganismen bzw. die Prüfung auf ausreichende Konservierung sind in der *USP* und im *Europäischen Arzneibuch* (*PharmEur*) beschrieben. Zur Kultivierung von *L. gasseri* wurde MRS-Medium (de Man et al., 1960: p. 1216 in Section 14, Bergey's Manual of Systematic Bacteriology, Vol. 2. Sneath et al. (eds.), Williams & Wilkins, London 1986) und Blutagar (nach Hackenthal & Bierkowski, 1951; modifiziert nach Lerche und Reuter: Zbl. Bakt. I. Abt. Orig. 182, 324-356, 1961) verwendet; Bebrütungstemperatur: 37°C. Das Wachstum von *L. gasseri* nach Ausplattieren auf MRS-Agar erfordert ein sauerstoff-armes Gasgemisch, wozu handelsübliche Anaerobiersysteme von *Oxoid* GmbH (Wesel, D) oder *Merck* KgaA (Darmstdt, D) hinreichend geeignet sind.

### d) Keimzahlbestimmung und Aktivitätsmessung:

Die Keimzahlen werden wie üblich über Koloniezählung (Kolonie bildende Einheiten = KBE) ermittelt und in Zehnerpotenzen wiedergegeben. Eine Aktivität von 100% ist definiert als die Keimzahl, die zu Beginn der Testung vorliegt. Die Abnahme von 2 Zehnerpotenzen kommt also einer Restaktivität von 1 % gleich. Eine solche Abnahme innerhalb einer definierten Frist von beispielsweise 28 Tagen (d) gilt für mikrobiologische Maßstäbe als akzeptabel. Eine signifikante Unterschreitung ist erst bei Abnahme um 3 Zehnerpotenzen (0,1% Restaktivität) gegeben. Für die Prüfung auf ausreichende Konservierung ist dagegen die rasche Inaktivierung der Testkeime innerhalb von wenigen Tagen eine wichtige Anforderung. Dem Fachmann ist geläufig, dass Vermehrung und Inaktivierung exponentiellen Raten entsprechen. Daher wird nachfolgend die Aktivität stets in Zehnerpotenzen dargestellt.

### e) Durchführung der Experimente:

Die Experimente wurden mit folgender Fragestellung durchgeführt:
1. Wie verhält sich die Aktivität der Testorganismen im jeweiligen Suspensionsmedium während 28 d bei Raumtemperatur.
2. Wie verhält sich die Aktivität der Testorganismen im jeweiligen Suspensionsmedium in Gegenwart von oligodynamisch wirkendem Ag⁺ während 28 d bei Raumtemperatur.
3. Wie verhält sich die Aktivität der Testorganismen im jeweiligen Suspensionsmedium im Gemisch mit probiotischer Kultur *L. gasseri* während 28 d bei Raumtemperatur.
4. Wie verhält sich die Aktivität der Testorganismen im jeweiligen Suspensionsmedium in Gegenwart von oligodynamisch wirkendem Ag⁺ und im Gemisch mit probiotischer Kultur *L. gasseri* während 28 d bei Raumtemperatur.

Die jeweiligen 100%-Aktivitäten (Start-Keimzahlen) sind in den Tabellen zu den Ergebnissen angegeben. Alle Testansätzen wurden jeweils von unbeimpften Kontrollen begleitet.

### Ergebnisse.

### Suspensionsmedium A:

Die Ergebnisse zu 1. und 2. (siehe e) zeigt die Tabelle 2.

**Tabelle 2:**

| Die Aktivität der Testkeime im Suspensionsmedium A mit und ohne Ag⁺ während 28 d bei Raumtemperatur (20-25°C). | | | | | | | |
|---|---|---|---|---|---|---|---|
| Testkultur | | Aktivität (KBE/ml) bei Probenahme (d) | | | | | |
| | | 0,d | 0,25 d | 1 d | 7 d | 14 d | 28 d |
| Kontrolle | - Ag⁺ | steril | n.t.*) | n.t. | n.t. | n.t. | steril |
| | + ·Ag⁺ | steril | n.t. | n.t. | n.t. | n.t. | steril |
| *E. coli* | - Ag⁺ | 1,2 × 10⁶ | 1,1 × 10⁶ | 7,4 × 10⁵ | 6,0 × 10⁸ | 5,8 × 10⁶ | 7,6 × 10⁷ |
| | + Ag⁺ | 1,0 × 10⁶ | 8,2 × 10⁵ | 2,6 × 10⁴ | 5,0 × 10⁷ | 6,4 × 10⁶ | 2,8 × 10⁵ |
| *Ps. aerug.* | - Ag⁺ | 1,8 × 10⁶ | 1,6 × 10⁶ | 5,0 × 10⁶ | 1,0 × 10⁹ | 6,0 × 10⁶ | 8,6 × 10⁶ |
| | + Ag⁺ | 2,0 × 10⁶ | 1,0 × 10⁶ | 4,4 × 10⁵ | 7,8 × 10⁶ | 3,6 × 10⁵ | 1,0 × 10⁶ |
| *St. aureus -* | Ag⁺ | 4,4 × 10⁵ | 5,0 × 10⁵ | 1,7 × 10⁸ | 7,9 × 10⁸ | 4,4 × 10⁶ | 7,8 × 10⁷ |
| | + Ag⁺ | 4,6 × 10⁵ | 8,2 × 10⁵ | 1,0 × 10⁶ | 9,6 × 10⁴ | 3,4 × 10³ | 4,8 × 10³ |
| *C. albicans -* | Ag⁺ | 1,1 × 10⁶ | 4,2 × 10⁵ | 1,2 × 10⁷ | 3,7 × 10⁷ | 7,0 × 10⁴ | 3,2 × 10⁵ |
| | + Ag⁺ | 6,8 × 10⁵ | 3,8 × 10⁵ | 4,2 × 10⁶ | 2,5 × 10⁷ | 2,4 × 10⁵ | 1,4 × 10⁵ |
| *A. niger* | - Ag⁺ | 5,4 × 10⁶ | 4,0 × 10⁷ | 1,2 × 10⁷ | Mycel**) | Mycel | Mycel |
| | + Ag⁺ | 1,5 × 10⁸ | 1,4 × 10⁸ | 7,4 × 10⁶ | Mycel | Mycel | Mycel |
| *L. gasseri* | - Ag⁺ | 6,0 × 10⁶ | n.t. | 4,6 × 10⁵ | 3,8 × 10⁶ | 2,2 × 10⁵ | 7,4 × 10⁵ |
| | + Ag⁺ | 6,0 × 10⁶ | n.t. | n.t. | 3,0 × 10⁶ | 1,2 × 10⁵ | 9,1 × 10⁴ |

| | | | | | | | |
|---|---|---|---|---|---|---|---|
| *) n.t. : nicht getestet; **) Mycelbildung aufgrund Zellzunahme, d. h. keine Hemmung. | | | | | | | |

Aus der Tabelle 2 ist zu entnehmen, dass in Medium A der oligodynamische Effekt von Ag⁺ aufgehoben ist. Das bedeutet, Medium A ist für eine Konservierung mittels Ag⁺ ungeeignet. Die gleiche Versuchsreihe unter Verwendung von Medium A, dem zusätzlich probiotischer *L. gasseri* in einer Menge von ca. 10⁶ KBE/ml zugesetzt war (gemäß 3. und 4. unter e), zeigte ebenfalls keinerlei konservierenden Effekt durch Ag⁺ (Ergebnisse nicht dargestellt).

### Suspensionsmedium B:

Die Ergebnisse zu 1. und 2. (siehe e) zeigt die Tabelle 3.

**Tabelle 3:**

| Die Aktivität der Testkeime im Suspensionsmedium B mit und ohne Ag⁺ während 28 d bei Raumtemperatur (20-25°C). | | | | | | | |
|---|---|---|---|---|---|---|---|
| Testkultur | | Aktivität (KBE/ml) bei Probenahme (d) | | | | | |
| | | 0 d | 0,25d | 1 d | 7d | 14d | 28 d |
| Kontrolle | - Ag⁺ | steril | n.t.*) | n.t. | n.t. | n.t. | steril |
| | + Ag⁺ | steril | n.t. | n.t. | n.t. | n.t. | steril |
| *E. coli* | - Ag⁺ | 5,6 × 10⁶ | 6,2 × 10⁶ | 7,2 × 10⁶ | 2,1 × 10⁷ | 1,1 × 10⁸ | 2,3 × 10⁹ |
| | + Ag⁺ | 2,4 × 10⁶ | 9,0 × 10⁵ | 5,4 × 10⁵ | < 20 | < 20 | < 20 |
| *St. aureus -* | Ag⁺ | 4,4 × 10⁶ | 3,8 × 10⁶ | 8,0 × 10⁵ | 1,6 × 10⁶ | 2,2 × 10⁹ | 7,6 ×10⁷ |
| | + Ag⁺ | 6,0 × 10⁶ | 4,8 × 10⁶ | 2,7 × 10⁵ | 4,6 × 10² | < 20 | < 20 |

| | | | | | | | |
|---|---|---|---|---|---|---|---|
| *) n.t. : nicht getestet; | | | | | | | |

Wie die Tabelle 3 zeigt, ist im Medium B in Gegenwart von Ag⁺ ein deutlicher oligodynamischer Effekt schon nach 1 d festzustellen (nur ein Teil der Testkeime geprüft). Die eigentliche Frage-stellung war jedoch, ob und welche Konservierungseigenschaften in Gegenwart von probiotischem *L. gasseri* zu beobachten sein würden (3. und 4. unter e). Dieses Ergebnis ist wie folgt aus Tabelle 4 zu entnehmen.

**Tabelle 4:**

| Die Aktivität der Testkeime im Suspensionsmedium B mit und ohne Ag⁺ und in Gegenwart von probiotischem *L. gasseri* (ca. 10⁶ KBE/ml) während 28 d bei 20-25°C. | | | | | | | |
|---|---|---|---|---|---|---|---|
| Testkultur | | Aktivität (KBE/ml) bei Probenahme (d) | | | | | |
| | | 0 d | 0,25 d | 1 d | 7 d | 14 d | 28 d |
| Kontrolle | - Ag⁺ | steril | n.t.*) | n.t. | n.t. | n.t. | steril |
| | + Ag⁺ | steril | n.t. | n.t. | n.t. | n.t. | steril |
| *E. coli* | - Ag⁺ | 4,8 × 10⁶ | 2,8 × 10⁵ | 3,6 × 10⁴ | 5,0 × 10³ | < 20 | < 20 |
| | + Ag⁺ | 3,8 × 10⁶ | 3,4 × 10⁵ | 4,4 × 10² | < 20 | < 20 | < 20 |
| *Ps. aerug.* | - Ag⁺ | 6,4 × 10⁶ | 3,6 × 10⁴ | 2,8 × 10⁵ | 7,2 × 10⁴ | < 20 | < 20 |
| | + Ag⁺ | 6,2 × 10⁶ | 7,0 × 10⁴ | 1,8 × 10² | < 20 | < 20 | < 20 |
| *St. aureus -* | Ag⁺ | 5,6 × 10⁶ | 1,3 × 10⁶ | 6,4 × 10⁵ | 1,8 × 10⁵ | 4,6 × 10³ | < 20 |
| | + Ag⁺ | 7,8 × 10⁶ | 4,2 × 10⁴ | 5,0 × 10² | < 20 | < 20 | < 20 |
| *C. albicans -* | Ag⁺ | 2,8 × 10⁶ | 4,4 × 10⁶ | 5,8 × 10⁶ | 2,4 × 10⁵ | 1,8 × 10³ | < 20 |
| | + Ag⁺ | 2,4 × 10⁶ | 6,4 × 10⁶ | 7,6 × 10⁵ | 3,2 × 10⁶ | 2,8 × 10⁵ | 7,6 × 10³ |
| *A. niger* | - Ag⁺ | 2,4 × 10⁶ | 1,4 × 10⁵ | 3,0 × 10⁶ | 6,2 ×10⁵ | 5,8 × 10⁴ | 2,8 ×10³ |
| | + Ag⁺ | 1,2 × 10⁷ | 7,8 × 10⁶ | 3,2 × 10⁵ | 1,4 × 10⁵ | 4,2 × 10⁵ | 2,4 × 10³ |
| *L. gasseri* | - Ag⁺ | 2,4 × 10⁶ | 2,4 ×10⁶ | 2,4 × 10⁶ | 1,2 × 10⁶ | 4,8 × 10⁵ | 4,6 × 10⁴ |
| | + Ag⁺ | 3,2 × 10⁶ | 2,8 × 10⁶ | 3,2 × 10⁵ | < 20 | < 20 | < 20 |

| | | | | | | | |
|---|---|---|---|---|---|---|---|
| *) n.t. : nicht getestet; | | | | | | | |

Überraschend zeigen die Aktivitäten in Tabelle 4 ein unerwartetes sicheres Konservierungsver-halten auch schon ohne Ag⁺, während umgekehrt die Aktivität der probiotischen Kultur nicht wesentlich geschwächt wird. Eine starke Abschwächung erfährt diese erst in Gegenwart von Ag⁺ (siehe Tabelle). Da im Dosiersystem jedoch der Silberfaden nicht im Gemisch (wie hier unter Testbedingungen) sondern im Auslassventil untergebracht ist, ist die oligodynamische Aktivitätsminderung von *L*. *gasseri* nur in diesem Teil des Systems und nicht im Suspensions-gemisch vorhanden.

Die konservierenden Eigenschaften des Suspensionsmediums, enthaltend probiotischen *L. gasseri,* übt also einen selbstkonservierenden Effekt aus, wobei die Aktivität von *L. gasseri* selbst bis zu 4 Wochen erhalten bleibt und so lange für Sprühstöße genutzt werden kann.

### Suspensionsmedium C:

Zum Vergleich mit Medium B testeten wir ein weiteres Medium, das jedoch durch einen 5mal höheren Pepton-Gehalt gekennzeichnet war. Wir wollten zunächst wissen, wie die selbstkon-servierende Wirkung, also in Abwesenheit von Ag⁺, davon beeinflußt wird.

**Tabelle 5: Die Aktivität der Testkeime im Suspensionsmedium C mit und ohne Ag⁺ während 28 d bei Raumtemperatur (20-25°C).**

| Testkultur | | Aktivität (KBE/ml) bei Probenahme (d) | | | | | |
|---|---|---|---|---|---|---|---|
| | | 0 d | 0,25 d | 1 d | 7 d | 14 d | 28 d |
| Kontrolle | - Ag⁺ | steril | n.t.*) | n.t. | n.t. | n.t. | steril |
| *E. coli* | - Ag⁺ | 1,0 × 10⁵ | n.t. | 4,0 × 10² | < 20 | < 20 | < 20 |
| *St. aureus -* | - Ag⁺ | 1,0 × 10⁵ | n.t. | 5,4 × 10⁴ | < 20 | < 20 | < 20 |

| | | | | | | | |
|---|---|---|---|---|---|---|---|
| *) n.t. : nicht getestet; | | | | | | | |

Wie die Tabelle 5 zeigt, ist im Medium C tatsächlich eine bemerkenswert und überraschend starke, selbstkonservierende Wirksamkeit zu beobachten. Es war daher besonders interessant zu prüfen, was für eine Aktivität *L. gasseri* und welchen Effekt Ag⁺ zeigen würden. Das Ergebnis dieser Testung zeigt Tabelle 6.

**Tabelle 6: Die Aktivität der Testkeime im Suspensionsmedium C mit und ohne Ag⁺ und in Gegenwart von probiotischem L. gasseri (ca. 10⁶ KBE/ml) während 28 d bei 20-25°C.**

| Testkultur (enthält *L. gasseri*) | | Aktivität (KBE/ml) bei Probenahme (d) | | | | | |
|---|---|---|---|---|---|---|---|
| | | 0 d | 0,25 d | 1 d | 7 d | 14 d | 28 d |
| Kontrolle | - Ag⁺ | steril | n.t.*) | n.t. | n.t. | n.t. | steril |
| | + Ag⁺ | steril | n.t. | n.t. | n.t. | n.t. | steril |
| *E. coli* | - Ag⁺ | 9,0 × 10⁵ | 3,8 × 10⁵ | 2,8 × 10⁴ | < 20 | < 20 | < 20 |
| | + Ag⁺ | 1,1 × 10⁶ | 8,4 × 10⁴ | 3,4 × 10³ | < 20 | < 20 | < 20 |
| *Ps. aerug.* | - Ag⁺ | 1,6 × 10⁵ | 7,2 × 10⁵ | 4,4 × 10⁴ | < 20 | < 20 | < 20 |
| | + Ag⁺ | 2,8 × 10⁶ | 5,0 × 10³ | < 20 | < 20 | < 20 | < 20 |
| *St. aureus -* | - Ag⁺ | 1,2 × 10⁶ | 5,6 × 10⁵ | 1,3 × 10⁶ | 4,6 × 10⁵ | 3,0 × 10³ | < 20 |
| | + Ag⁺ | 1,9 × 10⁶ | 2,4 × 10⁶ | 7,0 × 10⁶ | 2,2 × 10³ | < 20 | < 20 |
| *C. albicans -* | - Ag⁺ | 1,1 × 10⁷ | 4,2 × 10⁶ | 1,6 × 10⁷ | 2,8 × 10⁷ | 1,6 × 10⁵ | 4,6 × 10⁵ |
| | + Ag⁺ | 1,2 × 10⁷ | 6,8 × 10⁶ | 9,4 × 10⁶ | 2,0 × 10⁷ | 3,8 × 10⁶ | 2,4 × 10⁵ |
| *A. niger* | - Ag⁺ | 2,8 × 10⁵ | 3,8 × 10⁵ | 2,4 × 10⁵ | 1,8 × 10⁴ | 5,8 × 10⁴ | < 20 |
| | + Ag⁺ | 3,4 × 10⁵ | 4,0 × 10⁵ | 1,8 × 10⁴ | 2,2 × 10⁵ | 4,2 × 10³ | < 20 |
| *L. gasseri* | - Ag⁺ | 2,4 × 10⁶ | 2,8 × 10⁶ | 1,6 × 10⁶ | 1,7 × 10⁶ | 7,0 × 10⁵ | < 20 |
| | + Ag⁺ | 3,4 × 10⁶ | 2,8 × 10⁶ | 1,8 × 10⁶ | 2,8 × 10⁵ | < 20 | < 20 |

| | | | | | | | |
|---|---|---|---|---|---|---|---|
| *) n.t. : nicht getestet; | | | | | | | |

Auch Medium C weist ähnlich Medium B schon ohne Ag⁺ einen interessanten aktivitätssenkenden Effekt auf. im Vergleich mit Medium B ist aber der oligodynamische Effekt von Ag⁺, z. B. bei *Staphylococcus aureus* und bei *Lactobacillus gasseri,* erheblich vermindert - also einesteils ein ungünstiger und andernteils günstiger Effekt. Im Medium C, das 5mal mehr Pepton als Medium B enthält (siehe Tabelle 1), ist der oligodynamische Effekt leicht passiviert, jedoch nicht so vollständig wie in Medium A, das 10mal mehr Pepton enthält (vgl. Tabelle 3). Es ist somit offensichtlich, dass die oligodynamische Wirksamkeit von Ag⁺ von der Peptonmenge abhängt. Wenn dies richtig ist, müssten die Medien D und E, beide ohne Peptonzusatz, im Vergleichstest mit Medium B einen noch besseren oligodynamischen Effekt von Ag⁺ aufweisen. Dies wird im folgenden deutlich.

### Suspensionsmedien D und E:

Den Einfluß von Ag⁺ auf die Testkeime *E. coli, St. aureus* und *L. gasseri* zeigt Tabelle 7.

**Tabelle7:**

| Vergleich der Aktivität (in *log* KBE/ml) einiger Testkeime in den Suspensionsmedien B, D und E mit und ohne Ag⁺ bei Raumtemperatur (20-25°C). | | | | | | | | | | | | | |
|---|---|---|---|---|---|---|---|---|---|---|---|---|---|
| Testkultur | | Aktivität (log KBE/ml) bei Probenahme (d) und Medium | | | | | | | | | | | |
| | | 0 d | | | 0,25 d | | | 1 d | | | 7 d | | |
| | | **B** | **D** | **E** | **B** | **D** | **E** | **B** | **D** | **E** | **B** | **D** | **E** |
| Kontrolle | - Ag⁺ | st*) | st | st | n.t.*) | n.t. | n.t. | n.t. | n.t. | n.t. | st | st | st |
| | + Ag⁺ | st | st | st | n.t. | n.t. | n.t. | n.t. | n.t. | n.t. | st | st | st |
| *E. coli* | - Ag⁺ | 6,75 | 6,58 | 6,03 | 5,0 | 4,86 | 4,76 | 6,86 | 3,51 | -*) | 7,33 | - | - |
| | + Ag⁺ | 6,38 | 6,41 | 6,15 | 5,95 | 4,58 | 4,48 | 5,73 | - | - | - | - | - |
| *St. aureus -* | - Ag⁺ | 6,64 | 6,06 | 5,72 | 6,58 | 3,64 | 3,62 | 5,90 | 2,26 | - | 6,30 | - | - |
| | + Ag⁺ | 6,78 | 6,70 | 4,72 | 6,68 | 3,73 | 3,48 | 5,43 | - | - | 2,66 | - | - |
| *L. gasseri -* | - Ag⁺ | 6,70 | 6,68 | 6,62 | 6,80 | 6,58 | - | 6,60 | 6,36 | - | 6,0 | 5,80 | - |
| | + Ag⁺ | n.t. | n.t. | n.t. | n.t. | n.t. | n.t. | n.t. | n.t. | n.t. | n.t. | n.t. | n.t. |

| | | | | | | | | | | | | | |
|---|---|---|---|---|---|---|---|---|---|---|---|---|---|
| *) Abkürzungen und Symbole: n.t. : nicht getestet; st : steril; - : keine Aktivität mehr. | | | | | | | | | | | | | |

Die Ergebnisse in der Tabelle 7 sind nicht in Zehnerpotenzen sondern als dekadischer Logarithmus angegeben (die erste Ziffer entspricht numerisch der Zehnerpotenz). Die hier gezeigten Daten sind im Einklang mit der Annahme, dass der oligodynamische Effekt von Ag⁺ tatsächlich um so höher ist, je weniger Pepton im Medium, und dass die Testkeime in den Medien D und E auch ohne Ag⁺ schon rasch inaktiv werden. Allerdings hat das gänzliche Fehlen von Pepton auch negative Auswirkung, denn die Aktivität von *L*. *gasseri* wird ebenfalls rasch vermindert (so daß sich eine Testung von Ag⁺ im Gemisch erübrigte).

Aus den Ergebnissen lässt sich zusammenfassend sagen, daß überraschend ein Medium gefunden wurde, dass, angewendet in einem Dosierspray-System, zwar einerseits eine konservierende Wirkung gegen Verkeimung des Mediums besitzt, andererseits aber die Aktivität von im Gemisch enthaltenen probiotischen Milchsäurebakterien, zum Zweck des Sprühens auf die Haut oder Schleimhaut, ausreichend schützt.

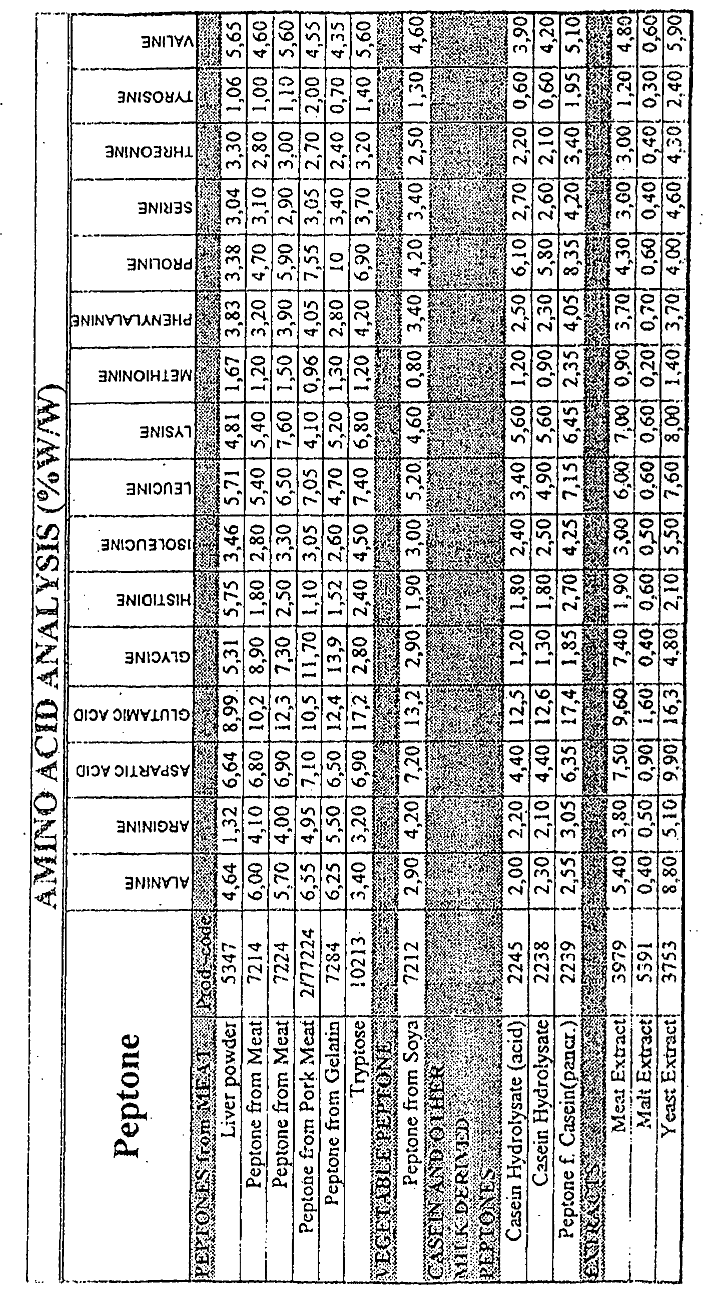

## Patentansprüche

1. Verwendung eines wässrigen Suspensionsmediums, das peptidische Bestandteile, insbesondere Pepton, und/oder wenigstens eine Aminosäure enthält, wobei das Supensionsmedium einen Gesamtstickstoffgehalt nach Kjeldahl von 0,01 bis 0,2% (m/v) hat, zur Aktivitätserhaltung und zur Konservierung von lebensfähigen Milchsäurebakterien für probiotische Anwendungen.

2. Verwendung nach Anspruch 1,
**dadurch gekennzeichnet,**
**dass** das Suspensionsmedium mit den Milchsäurebakterien auf Menschen, Tiere, Pflanzen oder Organe derselben aufgesprüht wird.

3. Zweikammer-Dosierabgabesystem, mit einer ersten Kammer, die in einem Ausgangszustand eine Suspensionsflüssigkeit enthält, einer zweiten Kammer, die in einem Ausgangzustand in trockener Form vorliegende Milchsäurebakterien enthält und luftdicht verschlossen ist, und mit einer Dosierpumpe, wobei vor Ingebrauchnahme des Systems die zweite Kammer zur ersten Kammer hin geöffnet wird, so daß die Milchsäurebakterien in die Suspensionsflüssigkeit gelangen, wobei die Suspensionsflüssigkeit eine oder mehrere Aminosäuren und/oder peptidische Bestandteile, insbesondere Pepton enthält, und wobei die Suspensionsflüssigkeit einen Gesamtstickstoffgehalt nach Kjeldahl von 0,01 bis 0,2% (m/v) hat.

4. Zweikammer-Dosierabgabesystem nach Anspruch 3,
**dadurch gekennzeichnet, daß** in dem Dosierkopf der Dosierpumpe in der Austrittsbahn der Abgabeflüssigkeit eine Sterilisationseinrichtung angeordnet ist, die Silber oder ein anderes oligodynamisch wirksames Metall enthält.

5. Zweikammer-Dosierabgabesystem nach wenigstens einem der Ansprüche 3 oder 4,
**dadurch gekennzeichnet, daß** die erste Kammer durch einen weichen Beutel gebildet ist, der sich bei Abgabe des Behälterinhalts zusammenzieht.

6. Zweikammer-Dosierabgabesystem nach wenigstens einem der Ansprüche 3 bis 5,
**dadurch gekennzeichnet, daß** die Dosierpumpe eine Airless-Pumpe ist, bei der verhindert ist, daß Umgebungsluft in die erste Kammer eintritt.

7. Zweikammer-Dosierabgabesystem nach wenigstens einem der Ansprüche 3 bis 6,
**dadurch gekennzeichnet, daß** der die erste Kammer bildende weiche Beutel in einem starren Außenbehälter angeordnet ist, in dessen Wand wenigstens ein Loch ausgebildet ist, durch das Luft zum Druckausgleich bei der Abgabe von Behälterinhalt in den Zwischenraum zwischen dem Außenbehälter und den weichen Beutel eintritt.

8. Zweikammer-Dosierabgabesystem nach wenigstens einem der Ansprüche 3 bis 4,
**dadurch gekennzeichnet, daß** die erste Kammer durch einen starren Behälter gebildet ist, in den nach Abgabe von Behälterinhalt jeweils Luft zum Druckausgleich eintritt, die dabei eine Filtereinrichtung durchströmt.

9. Zweikammer-Dosierabgabesystem nach wenigstens einem der Ansprüche 3 bis 8,
**dadurch gekennzeichnet, daß** die zweite Kammer durch einen rohrförmigen Einsatz gebildet ist, der in die erste Kammer hineinragt und in den der vordere Endabschnitt der Dosierpumpe eingreift, und daß der Boden der zweiten Kammer **dadurch** zu öffnen ist, daß die Dosierpumpe in axialer Richtung der Kammer vorgeschoben wird.

## Claims

1. The use of an aqueous suspension medium, which contains peptide components, particularly peptone, and/or at least one amino acid, wherein the suspension medium has a total nitrogen content in accordance with the Kjeldahl method of 0.01 to 0.2% (m/v) for maintaining the activity of, and for preserving, viable lactic acid bacteria for probiotic applications.

2. The use as claimed in Claim 1, **characterised in that** the suspension medium with the lactic acid bacteria is sprayed onto people, animals, plants or organs thereof.

3. A two-chamber metering dispensing system with a first chamber, which, in an initial condition, contains a suspension liquid, a second chamber which, in an initial condition, contains lactic acid bacteria in dry form and is hermetically sealed, and with a metering pump, wherein before the system is used, the second chamber is opened to the first chamber so that the lactic acid bacteria enter the suspension liquid, wherein the suspension liquid contains one or more amino acids and/or peptide components, particularly peptone, and wherein the suspension liquid has a total nitrogen content in accordance with the Kjeldahl method of 0.01 to 0.2% (m/v).

4. A two-chamber metering dispensing system as claimed in Claim 3, **characterised in that** disposed in the metering head of the metering pump in the outlet path of the delivery liquid there is a sterilisation device, which contains silver or another oligodynamically acting metal.

5. A two-chamber metering dispensing system as claimed in at least one of Claims 3 or 4, **characterised in that** the first chamber is constituted by a soft bag, which contracts when the container content is dispensed.

6. A two-chamber metering dispensing system as claimed in at least one of Claims 3 to 5, **characterised in that** the metering pump is an airless pump which prevents ambient air from entering into the first chamber.

7. A two-chamber metering dispensing system as claimed in at least one of Claims 3 to 6, **characterised in that** the soft bag constituting the first chamber is disposed in a rigid outer container, formed in the wall of which there is at least one hole, through which air enters for pressure balancing purposes into the space between the outer container and the soft bag when the contents of the container are dispensed.

8. A two-chamber metering dispensing system as claimed in at least one of Claims 3 to 4, **characterised in that** the first chamber is constituted by a rigid container, into which, after dispensing the contents of the container, air enters for pressure balancing purposes, which flows thereby through a filter device.

9. A two-chamber metering dispensing system as claimed in at least one of Claims 3 to 8, **characterised in that** the second chamber is constituted by a tubular insert, which projects into the first chamber and in which the front end section of the metering pump engages and that the base of the second chamber may be opened by advancing the metering pump in the axial direction of the chamber.

## Revendications

1. Utilisation d'un milieu de suspension aqueux contenant des composants peptidiques, en particulier de la peptone et/ou au moins un aminoacide, le milieu de suspension ayant une teneur en azote total suivant Kjeldahl de 0,01 à 0,2% (m/V), pour le maintien de l'activité et pour la conservation de bactéries de l'acide lactique viables pour des applications probiotiques.

2. Utilisation selon la revendication 1, **caractérisée en ce que** le milieu de suspension contenant les bactéries de l'acide lactique est pulvérisée sur des êtres humains, des animaux, des plantes ou des organes des précédents.

3. Système de délivrance dosée à deux chambres, avec une première chambre qui dans un état initial contient un liquide de suspension, avec une deuxième chambre qui dans un état initial contient des bactéries de l'acide lactique présentes sous forme séchée et qui est fermée de manière étanche à l'air, et avec une pompe doseuse, dans lequel, avant la mise en service du système, la deuxième chambre est ouverte en direction de la première chambre de façon que les bactéries de l'acide lactique parviennent dans le liquide de suspension, ledit liquide de suspension contenant un ou plusieurs aminoacides et/ou composants peptidiques, en particulier de la peptone, et le liquide de suspension ayant une teneur en azote total suivant Kjeldahl de 0,01 à 0,2% (*m*/*V*).

4. Système de délivrance dosée à deux chambres selon la revendication 3, **caractérisé en ce qu'**un dispositif de stérilisation contenant de l'argent ou un autre métal ayant une activité oligodynamique est disposé dans la tête de dosage de la pompe doseuse dans le trajet de sortie du liquide délivré.

5. Système de délivrance dosée à deux chambres selon l'une au moins des revendications 3 ou 4, **caractérisé en ce que** la première chambre est formée par une poche souple qui se contracte lors de la délivrance du contenu du récipient.

6. Système de délivrance dosée à deux chambres selon l'une au moins des revendications 3 à 5, **caractérisé en ce que** la pompe doseuse est une pompe airless dans laquelle l'air ambiant est empêché de pénétrer dans la première chambre.

7. Système de délivrance dosée à deux chambres selon l'une au moins des revendications 3 à 6, **caractérisé en ce que** la poche souple qui forme la première chambre est disposée dans un récipient extérieur rigide dans la paroi duquel est formé au moins un trou par lequel de l'air servant à égaliser la pression pénètre dans l'espace intermédiaire entre le récipient extérieur et la poche souple lors de la délivrance du contenu du récipient.

8. Système de délivrance dosée à deux chambres selon l'une au moins des revendications 3 à 4, **caractérisé en ce que** la première chambre est formée par un récipient rigide dans lequel de l'air pénètre après chaque délivrance du contenu du récipient afin d'égaliser la pression, en traversant pour cela un dispositif filtrant.

9. Système de délivrance dosée à deux chambres selon l'une au moins des revendications 3 à 8, **caractérisé en ce que** la deuxième chambre est formée par un insert tubulaire qui dépasse dans la première chambre et pénètre dans la section d'extrémité avant de la pompe doseuse et **en ce que** le fond de la deuxième chambre est ouvert en faisant avancer la pompe doseuse dans le sens axial de la chambre.
